Europäisches Patentamt

European Patent Office

Office européen des brevets

(19)

(11) EP 1 475 090 A1

(12) **EUROPEAN PATENT APPLICATION**
published in accordance with Art. 158(3) EPC

(43) Date of publication:
10.11.2004 Bulletin 2004/46

(21) Application number: 03705087.9

(22) Date of filing: 13.02.2003

(51) Int Cl.7: **A61K 31/198**, A61K 9/08,
A61K 9/14, A61K 9/16,
A61K 9/20, A61P 3/10,
A61P 25/00, A61P 25/10,
A61P 25/14, A61P 25/16,
A61P 25/28, A61P 27/02,
A61P 43/00

(86) International application number:
PCT/JP2003/001462

(87) International publication number:
WO 2003/068215 (21.08.2003 Gazette 2003/34)

(84) Designated Contracting States:
AT BE BG CH CY CZ DE DK EE ES FI FR GB GR
HU IE IT LI LU MC NL PT SE SI SK TR
Designated Extension States:
AL LT LV MK RO

(30) Priority: 14.02.2002 JP 2002036468

(71) Applicant: Ajinomoto Co., Inc.
Tokyo 104-8315 (JP)

(72) Inventors:
• MORI, Maiko c/o Ajinomoto Co., Inc.
Kawasaki-shi, Kanagawa 210-0801 (JP)

• TAKAHARA, Yoshiyuki c/o Ajinomoto Co., Inc.
Kawasaki-shi, Kanagawa 210-0801 (JP)
• ISHIZAKI, Sonoko c/o Ajinomoto Co., Inc.
Kawasaki-shi, Kanagawa 210-0801 (JP)
• SONAKA, Ichiro c/o Ajinomoto Co., Inc.
Kawasaki-shi, Kanagawa 210-0801 (JP)

(74) Representative: Nicholls, Kathryn Margaret et al
Mewburn Ellis LLP
York House
23 Kingsway
London WC2B 6HP (GB)

(54) **DRUGS FOR MITOCHONDRIAL DISEASES**

(57) A novel pharmaceutical agent effective for preventing, ameliorating, and/or therapeutically treating a series of diseases generically calledmitochondrial disorders is provided. Alanine such as L-alanine is used as an active ingredient in the objective pharmaceutical agent. Providing also a method for preventing, ameliorating and/or therapeutically treating mitochondrial disorders together with the use of alanine in producing the pharmaceutical agent for mitochondrial disorders.

EP 1 475 090 A1

## Description

Technical Field

The present invention relates to a pharmaceutical agent for preventing, ameliorating and therapeutically treating mitochondrial disorders, namely a pharmaceutical agent for mitochondrial disorders (medical products such as prophylactic agent, ameliorating agent and therapeutic agent). Further, the inventionrelates toamethodforpreventing, amelioratingand/or therapeutically treating mitochondrial disorders, the use of alanine such as L-alanine in producing a pharmaceutical agent for mitochondrial disorders and the like. Mitochondrial disorders are the generic name of a series of pathological states occurring due to the functional abnormality of mitochondria. The pharmaceutical agent for mitochondrial disorders in accordance with the invention contains alanine such as L-alanine as one of amino acids as the active ingredient.

Related Art

Mitochondrial disorders are the generic name of a series of diseases with the etiology of the functional disorder of mitochondria. Mitochondria have the function of energy generation in cells. It is known that when the function is deteriorated, various diseases are induced. Examples of those diseases include MELAS, Leigh's encephalopathy, Huntington disease, Parkinson disease, Alzheimer's disease, MNGIE, chronic progressive external ophthalmoplegia (CPEO), MERRF syndrome, and Leber's disease. It is considered that the diseases are derived from functional abnormalities of various metabolic enzymes existing in mitochondria. It is inferred that the etiology resides in the mutation of the corresponding genes.

Mitochondrial disorders are degenerative diseases due to various mechanisms such as abnormality of mitochondrial DNA (deletion, point mutation, and duplication), abnormality of cellular DNA encoding mitochondrial enzymes or complex polymeric mitochondrial components, and acquired one induced by toxic substances or pharmaceutical products. When mitochondria-associated genes are damaged because of these reasons, various biochemical abnormalities occur. In other words, carnitine palmitoyl transf erase deficiency and carnitine deficiency due to substrate transfer disturbances, pyruvate carboxylase deficiency and pyruvate dehydrogenase complex deficiency due to disturbances in substrate use, $\beta$-oxidation disturbance, fumarase deficiency and $\alpha$-ketoglutarate dehydrogenase deficiency due to disturbance in the TCA cycle, Luff disease due to disturbance of oxidative phosphorylation conjugation, complex I deficiency, complex II deficiency, complex III deficiency and complex IV (cytochrome c oxidase) deficiency and complex V (ATP synthase) deficiency due to electron transfer enzyme damages and the like. The essential etiology of these disorders is the mitochondrial energy metabolic disorders. Therefore, the therapeutic methods thereof include therapeutic treatments of the genes as the cause of the disorders, methods for supplementing the enzymes, substrate controls for the damaged enzymes, and making toxic substances non-toxic, or supplementation of lacking substances. Specific therapeutic agents include coenzyme $Q_{10}$, vitamins, cytochrome formulations, succinic acid, dichloroacetic acid, carnitine, and ATP formulations. Currently, no definite therapeutic agents or therapeutic methods exist.

Disclosure of the Invention

1. Problem to be solved by the present invention

The problem that the invention is to solve is to provide a novel pharmaceutical agent effective for the prophylaxis, amelioration and therapeutic treatment of a series of diseases generically called mitochondrial disorders.

2. Means for Solving the Problem

To overcome the problem, the present inventors first examined and evaluated the related art.

Because cardiochrome as a complex of cytochrome c with vitamins $B_1$ and $B_2$ is prepared from bovine myocardium, potentially, cytochrome c may cause anaphylaxis shock. Additionally, although it has been expected to supplement substrates for cytochrome c oxidase, the effectiveness of the agent is low, when the residual activity of the enzyme is low. Oral cytochrome formulations such as Cytorest are also on market, however, a sufficient amount of cytochrome may possibly not be delivered in mitochondria when administered orally. Alternatively, it was assumed that alanine might overcome these problems. Thus, an attempt was made to administer L-alanine. It was then confirmed that the mRNA expression of cytochrome c oxidase increased. Further, cytochrome c oxidase itself was activated. Therefore, the effectiveness will possibly be obtained even in a patient with a low residual activity of the enzyme. Additionally, oral alanine administration possibly activates the enzyme, involving no risk of anaphylaxis.

Additionally, dichloroacetic acid activates the TCA cycle by enhancing the activity of pyruvate dehydrogenase.

When the dose is high, however, side effects such as disordered consciousness occur. In contrast, alanine enhances the mRNA expression of isocitrate dehydrogenase, 2-oxoglutarate carrier, and fumarate hydratase, to activate the TCA cycle with no risk of side effects.

Next, ATP formulations are used for the purpose of supplementing intracellular ATP. Because ATP is readily decomposed, the effect is unknown. In contrast, alanine promotes the activity of ATP synthase by enhancing the mRNA expression of ATP synthase, to increase the ATP amount. Because ATP is generated intracellularly, alanine may possibly be more effective.

Alternatively, succinic acid is the substrate of the complex II. However, it is unknown whether or not succinic acid is sufficiently delivered in mitochondria via its administration. In contrast, alanine administration increases the mRNA expression of isocitrate dehydrogenase and 2-oxoglutarate carrier in the TCA cycle. This can activate isocitrate dehydrogenase in the TCA cycle, to promote succinate generation from 2-oxoglutaric acid. Because succinic acid is generated intracellularly, alanine may possibly be more effective.

Based on the results from the above examination and evaluation, the present inventors found that alanine such as L-alanine as one of amino acids activated the TCA cycle and electron transfer system as the important mitochondrial physiological functions and that based on the functions, the pathological state of mitochondrial disorders was prevented, ameliorated and therapeutically treated and the like. Thus, the invention has been achieved on the basis of the finding.

The invention relates to a pharmaceutical agent for use in the prophylaxis, amelioration and therapeutic treatment of mitochondrial disorders (referred to as "pharmaceutical agent for mitochondrial disorders"), the pharmaceutical agent containing alanine such as L-alanine as the active ingredient.

The steric configuration of alanine for use in accordance with the invention has not specific limitation. L-form and D-form thereof can be used. For simplicity, L-alanine can be used.

The mitochondrial disorders include for example any pathological states of MELAS, Leigh's encephalopathy, Huntington disease, Parkinson disease, Alzheimer's disease, MNGIE, chronic progressive external ophthalmoplegia (CPEO), MERRF syndrome, Leber's disease and diabetes mellitus.

The dose of the active ingredient alanine of the pharmaceutical agent of the invention is preferably about 10 to 1,000 mg/kg per day, more preferably about 100 to 500 mg/kg per day, still more preferably about 200 to 400 mg/kg per day and can be given into a biological organism requiring the pharmaceutical agent.

The dosage form of the pharmaceutical agent of the invention preferably includes for example but is not limited to tablets, granules, powders, and injections.

In another aspect, the invention relates to a method for preventing, ameliorating and/or therapeutically treating mitochondrial disorders comprising a step of ingesting and administering alanine such as L-alanine to a biological organism. As the form for the ingestion or administration, various forms of the pharmaceutical agent for mitochondrial disorders in accordance with the invention can be selected.

In an additional aspect, the invention relates to the use of alanine such as L-alanine in producing a pharmaceutical agent for mitochondrial disorders. As to the form of such alanine for use in producing the pharmaceutical agent (the form for use after production), various forms of the pharmaceutical agent for mitochondrial disorders in accordance with the invention can be used.

Brief Description of Drawings

Fig. 1 depicts the results of the analysis of gene expression (gene expression of mitochondrial ATP generation-associated protein) in Example 1; where open square (□) expresses group 2 (control group) and solid square (■) expresses group 1 (Ala-dosed group).

Fig. 2 depicts the results of the measurement of mitochondrial membrane potential in Example 2; and the influence of L-alanine on mitochondrial membrane potential (JC-1).

Fig. 3 depicts the results of the measurement of COX activity in Example 3; and the influence of L-alanine on the COX activity.

Mode for Carrying out the Invention

The mode for carrying out the invention is described below.

The invention relates to a pharmaceutical agent using alanine such as L-alanine as the active ingredient, which is a pharmaceutical agent to be administered into animals, particularly humans, namely biological organisms for the prophylaxis, amelioration and therapeutic treatment of mitochondrial disorders.

Via the administration of alanine, particularly L-alanine into biological organisms, the following ameliorating actions for mitochondrial disorders are suggested.

1. Via the administration of L-alanine, the mRNA expression of isocitrate dehydrogenase, 2-oxoglutaric acid carrier and fumarate hydratase in the TCA cycle can be increased. This activates the TCA cycle topromoteATP synthesis. In otherwords, it ameliorates mitochondrial disorders with disorders in the ATP synthesis, such as MELAS, MERRF syndrome, Leigh's encephalopathy, Parkinson disease, Alzheimer's disease, Huntington disease, Leber's disease, chronic progressive external ophthalmoplegia (CPEO), diabetes mellitus and MNGIE.

2. Via the administration of L-alanine, the mRNA expression of isocitrate dehydrogenase and 2-oxoglutaric acid carrier in the TCA cycle is increased. This activates the isocitrate dehydrogenase in the TCA cycle to promote succinate generation from 2-oxoglutaric acid. Specifically, the supply of succinic acid as the complex II substrate in the electron transfer system supplements ATP generation from the complex II in mitochondrial disorders with the deficiency of the complex I or the reduction of the activity of the complex I, particularly chronic progressive external ophthalmoplegia (CPEO), Leigh's encephalopathy, Parkinson disease, Alzheimer disease, Huntington disease, Leber's disease and the like, to ameliorate the pathological states thereof.

3. The administration of L-alanine increases the mRNA expression of cytochrome c oxidase in the electron transfer system. Specifically, cytochrome c oxidase is activated. L-Alanine promotes the increase of the activity of cytochrome c oxidase in the Leigh' s encephalopathyandKearns-Sayre syndrome with diffused reduction of cytochrome c oxidase activity, so that the pathological states thereof can be ameliorated.

4. The administration of L-alanine increases the mRNA expression of ATP synthase in the electron transfer system. In other words, it accelerates the activity of ATP synthase. Consequently, L-alanine accelerates ATP synthesis in diabetes mellitus due to insulin secretion disorders because of the functional deterioration of mitochondria and the reduction of ATP generation, so that insulin secretion is resumed. Additionally, L-alanine accelerates ATP synthesis in mitochondrial disorders with disordered ATP synthesis, such as MELAS, MERRF syndrome, Leigh's encephalopathy, Parkinson disease, Alzheimer's disease, Huntington disease, Leber's disease, chronic progressive external ophthalmoplegia (CPEO) and MNGIE, so that the pathological states thereof can be ameliorated.

In accordance with the invention, a rat after partial 70% hepatectomy as an animal model of liver disorders was used to confirm the efficacy of alanine, particularly L-alanine (see the following Examples). The animal model was reported to have reduced mitochondrial functions.

The dosage form and dosing form of the pharmaceutical agent of the invention are with no specific limitation. Therefore, various dosing forms can be adopted, including for example oral administration and parenteral administration (intravenous administration and the like, intraperitoneal administration, transdermal administration, inhalation administration, infusion administration and the like). For example, tablets, granules, powders, and injections can be adopted as the dosage forms.

The dose of the pharmaceutical agent of the invention can appropriately be selected, depending on the type (pathological states) of a mitochondrial disorder and the severity of the symptoms, and the form (dosage form) of the formulation. In case that alanine, for example L-alanine as the active ingredient is to be administered orally, a dose of L-alanine is preferably about 10 to 1000 mg/kg, more preferably about 100 to 500 mg/kg, andmostpreferably about 200 to 400 mg/kg per day per such patient. In case of a severe case, the dose can be increased more. Concerning the number and timing of dosing, the dose can be administered once in several days or once daily. Generally, the dose can be divided in several portions, for example 2 to 4 portions for sustained administration.

In case of parenteral dosing, alternatively, the dose can be selected and administered on the basis of a dose of about 1/10- to 1/20-fold the dose for oral dosing as described above.

The dose calculation is carried out in a manner corresponding to the range of the dose of alanine such as L-alanine contained in the pharmaceutical agent of the invention. The amount of alanine, particularly L-alanine derived from food and drinkproducts, nutrition, and other medical products as supplied through a route different from the administration route of the pharmaceutical agent to animals, particularly patients to be administered with the pharmaceutical agent is never included in the calculation of the dose. Therefore, a patient with a mitochondrial disorder or the like to which the pharmaceutical agent is to be administered can reasonably ingest L-alanine separately from the object of the present invention, for example in the daily dietary life.

Additionally, the formulation may contain various pharmacologically acceptable substances for formulation (as auxiliary agents and the like). A substance for formulation can be selected, appropriately, depending on the dosage form of the formulation. For example, the substances include excipients, diluents, additives, disintegrators, binders, coating agents, lubricants, sliding agents, lubricating agent, flavors, sweeteners and solubilizers. Further, the substances for formulation are specifically listed as follows: magnesium carbonate, titanium dioxide, lactose, mannitol and other sugars, talc, milk protein, gelatin, starch, cellulose and derivatives thereof, animal and vegetable oils, polyethylene glycol, and solvents, for example sterilized water and monohydric and polyhydric alcohols, for example glycerol.

Forms of these various medical formulations include for example appropriate solid or liquid formulation forms, for example granules, powders, coated tablets, tablets, (micro) capsules, suppositories, syrup, juice, suspensions, emulsions, drops, injection solutions and formulations for sustained release of the active substance.

The pharmaceutical agent of the invention in the form of the formulation listed above should reasonably contain the ingredient alanine at an amount effective for the exertion of the pharmaceutical efficacy. With reference to the dose and the like, the active ingredient can be blended in the pharmaceutical agent.

In another aspect as described above, the invention relates to a method for preventing, ameliorating and/or therapeutically treating mitochondrial disorders, including a step of allowing biological organisms to ingest alanine such as L-alanine or administering alanine such as L-alanine to biological organisms. In an additional aspect, the invention relates to the use of alanine such as L-alanine in producing a pharmaceutical agent for mitochondrial disorders.

All these inventions, particularly including the ingestion or administration form or the form for the use can be carried out readily with reference to the descriptions about the pharmaceutical agent for mitochondrial disorders in accordance with the invention and descriptions about the following Examples and the like and additionally with reference to the related art if necessary.

Preferable Mode for Carrying out the Invention

The invention is now specifically described in the following Examples. However, the invention is not limited to the Examples.

(Example 1)

Expression of the genes for mitochondrial ATP generation-associated proteins

After male F344/DvCrj (Fischer) rats (age 6 weeks and body weightof 120g) werepreliminarilyfedwitha feed CRF-1 (Oriental Yeast) and water for 6 weeks, the rats were divided in two groups of equal body weight. According to the Higgins-Anderson's method (see Higgins, G.M. and Anderson, R.M., 1931, Arch. Pathol. 12: 186-191), partial 70% hepatectomy (left lobe, intermediate lobe) was done. 18 and 21 hours after the partial hepatectomy, L-alanine at 2 g/10 ml/kg BW in aqueous 0.3% carboxymethyl cellulose solution was orally given (group 1), while only the aqueous 0.3% carboxymethyl cellulose solution was given as a control (group 2). 24 hours after the partial hepatectomy, anatomy wasdone. After killing under bleeding with anesthesia, liver was resected and weighed, and was then frozen and stored for assaying mRNA expression by the Taqman-PCR method.

10 ml ISOGEN (Nippon Gene) was added per 1 g of rat liver, for homogenization. The resulting homogenate was centrifuged to recover the supernatant. Chloroform at 200 $\mu$l per 1 ml of ISOGENwas added to the supernatant, forgentleagitation. After the resulting mixture was left to stand at ambient temperature for 2 minutes, the mixture was centrifuged at 15,000 rpm and 4°C for 10 minutes, to recover the aqueous layer. To the aqueous layer was added an equal volume of 2-propanol, and the resulting mixture was left to stand at ambient temperature for 5 minutes and centrifuged at 15,000 rpm and 4°C for 15 minutes. After the supernatant was discarded, 70% ethanol was added to the precipitated pellet. The resulting mixture was centrifuged at 15,000 rpm and 4°C for 15 minutes. The pellet was recovered. The pellet was dried at ambient temperature for 5 minutes, to which was added DEPC (diethylpyrocarbonate)-treated water, to solubilize the pellet.

The synthesis of template cDNA for use in the Taqman PCR was carried out, using SuperScript First-Strand Synthesis System for RT-PCR (manufactured by GIBCO BRL). 500 ng of total RNA, 1 $\mu$l of 0.5 $\mu$g/$\mu$l Oligo (dT)$_{12\text{-}18}$, and 1 $\mu$l of 10 mM dNTP mix were dissolved in the DEPC-treated water, to a final volume of 10 $\mu$l. After reaction at 65°C for 5 minutes, the reaction mixture was cooled with ice, followed by addition of 2 $\mu$l of 10 × RT buffer, 4 $\mu$l of 25 mM MgCl$_2$, 2 $\mu$l of 0.1 M DTT, and 1 $\mu$l of RNase Inhibitor. The resulting mixture was mixed together and then kept warm at 42°C for 2 minutes, to which a reverse transcriptase SUPERSCRIPT II RT of 1 $\mu$l (50 units) was added. The mixture was reacted together at 42°C for 50 minutes and then at 70°C for 15 minutes.

Primers for the proteins involved in the mitochondrial TCA cycle and the ATP generation in the electron transfer system were designed. For the designing, an outside database Primer 3 was used. (http://www.genome.wi.mit.edu/cgi-bin/primer/primer3_www.cg i)

Table 1 shows the types of the genes and the Unigene Nos. (http://www.ncbi.nlm.nih.gov/Unigene), and the nucleotide sequences of the primers (see SEQ ID Nos. 1-26 in the Sequence listing).

[Table 1]

| Unigene NO.(Rn) | Name of gene | Primer sequence | |
|---|---|---|---|
| | | 5' terminus | 3' terminus |
| 1093 | Rattus norvegicus mRNA for NAD+ specific isocitrate dehydrogenase b-subunit, partial cds | tca gct tcc aac atg cta cg | ctt gcc aac ctt gat cac ct |
| 2837 | NAD(H)-specific isocitrate dehydrogenase gamma subunit | tgg gtg atg gac tct tcc tc | gct gca ttg ttg tgt tgt cc |
| 29782 | Fumarate hydratase | gcc aag act gca cac aag aa | ctt ggg ttt cac cca ctc at |
| 880 | Cytochrome c oxidase subunit VIa (liver) | tgg acc ctg act ctt gtg tg | aag gga tgg agg agc aaa gt |
| 1745 | Cytochrome c oxidase subunit VIIa 3 | gtt cag tag tcg cgg ttg gt | gaa gtg gtg ctg atg gtc ct |
| 2270 | Rattus norvegicus liver cytochrome c oxidase subunit VIII (COX-VIII) mRNA, 3' end of cds | ttc ctg ctt cgt gtg ttg tc | tca aag gat gag gga aga cg |
| 19207 | Rattus norvegicus mRNA for cytochrome C oxidase assembly protein COX17, complete cds | tgt gcg atc gtt act gct tt | agg gct tca gag gct tct tc |
| 10249 | Cytochrome b5, outer mitochondrial membrane isoform | cca tcg tgg gtg cta ttc tt | agg aga tgt gct ccg aca ct |
| 80 | ATP synthase subunit d | aac gct ctg aag tcc tgg aa | gtc cac att ggc cct gta gt |
| 3357 | ATP synthase, H+ transporting, mitochondrial F0 complex, subunit c (subunit 9), isoform 1 | aaa aat gca gac cac gaa gg | cta ctc agg agg gag gca ga |
| 9723 | Rattus norvegicus (clone gamma-3) ATP synthase gamma-subunit (ATP5c) mRNA, 3' end cds | gct gac gtg ctg cag aat ta | atc ctg gca ctc tgc tca ct |
| 853 | 2-oxoglutarate carrier | ttt ctt cag cct gtg gaa gg | gac gct gtt agg cct tgt tc |
| 69 | β -actin | aaa tgc ttc tag gcg gac tg | aaa gcc atg cca aat gtc tc |

The reaction mixture of the composition shown below in Table 2 was mixed together in a PCR tube for Taqman, and the PCR reaction was carried out with ABI7700 Prism Sequence Detector. The reaction conditions were as follows.

Reaction conditions:

A cycle composed of 50°C for 2 minutes → 95°C for 10 minutes → (95°C for 15 seconds → 60°C for 1 minute) was carried out 40 times.

[Table 2]

| Composition of PCR reaction mixture (per tube) Template cDNA (corresponding to 2.5 ng of total RNA) | 0.1 μl |
|---|---|
| 5 units/μl AmpliTaq Gold | 0.05 μl |
| DNTP mix (each 2.5 mM) | 0.8 μl |
| 25 mM MgCl$_2$ | 1.2 μl |
| 10 × SYBR buffer | 1 μl |
| Primer solution (5' terminus) | 1 μl |
| Primer solution (3' terminus) | 1 μl |
| DH$_2$O | total 10 μl |

All the Taqman PCR reactions were carried out in a duplicate manner, to calculate the cycle time (CT) exceeding the threshold. The expression level at CT = 30 was defined as 1. By the following formula, the relative expression level was determined.

$$\text{Relative expression level} = 2^{(30-CT\ sample)}$$

Further, the relative value of the expression level of each gene was determined when the relative expression level of β-actin was defined as 1000.

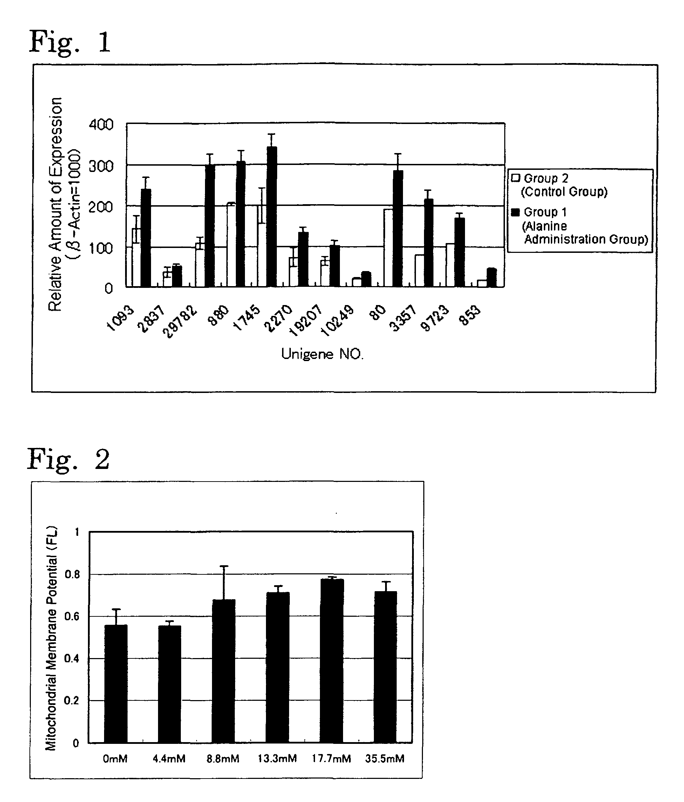

The results of the analysis of gene expression are shown in Fig. 1. As apparently shown in Fig. 1, L-alanine administration significantly increased themRNAs of theproteins involved in the TCA cycle of liver mitochondria and the ATP generation in the electron transfer system, compared with the non-administered group. Thus, it was shown that L-alanine had an effect of improving the energy generation in liver mitochondria.

(Example 2)

Assaying mitochondrial membrane potential

After a male SD (IGS) rat of age 8 weeks was killed under bleeding with ether anesthesia, liver was resected and washed with 1× Extraction Buffer A (10 mM HEPES (2-[4-(2-hydroxyethyl)-1-piperazinyl]ethanesulfonic acid, pH 7.5, 200 mM mannitol, 70 mM sucrose, 1 mM EGTA (ethylene glycol bis(β-aminoethyl ether) -N, N,N,N-tetraacetic acid) two times. The liver was cut into a piece of about 100 mg and weighed, which was then cut into smaller pieces in a 5-ml tube.

A 10-fold volume of 1× Extraction Buffer A (containing 2 mg/ml albumin) was placed in the tube and homogenized at a low speed in ice (15 seconds). The resulting homogenate was transferred in an Eppendorf tube, for centrifugation at 600 g at 4°C for 5 minutes. The supernatant was transferred in a new tube, for centrifugation at 11,000 g and 4°C for 10 minutes. After the supernatant was discarded, the precipitate was suspended in a 10-fold volume of 1× Extraction Buffer A. The procedure from centrifugation at 600 g and 4°C for 5 minutes to centrifugation at 11, 000 g and 4°C for 10 minutes was again repeated. The resulting supernatant was discarded. The precipitate was suspended in 40 μl of a storage buffer (10 mM HEPES pH 7.4, 250 mM sucrose, 1 mM ATP, 0.08 mM ADP (adenosine-5'-diphosphate), 5 mM sodium succinate, 2 mM $K_2HPO_4$, 1 mMDTT). The resul ting suspension was defined as mi tochondrial suspension for use in the following experiments. Through protein assay, the mitochondrial suspension was adjusted to 1 mg/ml. 2 μl (2 μg) of the mitochondrial suspension was mixed with 190 μl of an assay buffer (20 mM MOPS (3-(N-morpholino)propanesulfonic acid) , pH 7.5, 100 mM KCl, 10 mM ATP, 10 mM $MgCl_2$, 10 mM sodium succinate, 1 mM EGTA) , to which various concentrations of L-alanine were added. 2 μl of JC-1 (1 μg/ml) was added and left to stand at ambient temperature for 7 minutes, for assaying fluorescence intensity. (Excitation at 490 nm; emission at 590 nm) The results are shown in Fig. 2. Consequently, it was shown that the fluorescence intensity of JC-1 was increased in a manner dependent on the concentration of L-alanine added. This suggests that the electron transfer system of mitochondria is activated to increase the membrane potential.

The results described above indicate that L-alanine is promising as an active ingredient for pharmaceutical agents for mitochondrial disorders.

(Example 3)

Assaying COX (cytochrome C oxidase) activity

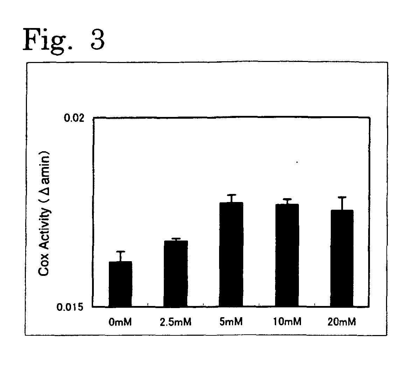

In the same manner as in Example 2, a mitochondrial suspension was prepared and adjusted to 0.1 mg/ml. 25 μl (2.5 μg) of mitochondria was blended in 950 μl of an assay buffer (10 mM Tris-HCl, pH 7.0, 120 mM KCl), to which various concentrations of L-alanine were added. 50 μl of cytochrome c (0.22mM) reduced with 10 mg/ml sodium hydroxysulfite was added, and the resulting mixture was inversed and mixed together to start reaction. Immediately, the absorbance (550 nn) was counted continuously for one minute with a spectrophotometer, to record the decrease of the absorbance. The results are shown in Fig. 3. It was shown that L-alanine had an action of increasing the COX activity in a concentration-dependent manner from 2.5 mM. It was suggested that the activation of the COX activity was partially involved in the activation of the mitochondrial membrane with L-alanine in Example 2.

The results described above show that alanine, particularly L-alanine can be used as an active ingredient for pharmaceutical agents for mitochondrial disorders.

Advantages of the Invention

The invention can provide a pharmaceutical agent for example a therapeutic agent effective for mitochondrial disorders. Additionally, the pharmaceutical agent for mitochondrial disorders in accordance with the invention contains the amino acid as the active ingredient, so the pharmaceutical agent is highly safe with almost no adverse effects.

Thus, the pharmaceutical agent is extremely useful as a medical product for mitochondrial disorders.

Further, theinventioncanprovideamethodforpreventing, ameliorating and/or therapeutically treating mitochondrial disorders and the use of alanine such as L-alanine in producing pharmaceutical agents for mitochondrial disorders.

Therefore, the invention is widely applicable to fields of medical products, food products and medicine. Accordingly, the invention is very useful industrially.

**Claims**

1. A pharmaceutical agent for mitochondrial disorders, the pharmaceutical agent comprising alanine as an active ingredient.

2. The pharmaceutical agent according to claim 1, wherein said alanine is L-alanine.

3. The pharmaceutical agent according to claim 1 or 2, the pharmaceutical agent being an agent for preventing, ameliorating and/or therapeutically treating mitochondrial disorders.

4. The pharmaceutical agent according to claim 1 or 2, wherein the pharmaceutical agent is used for any of MELAS, Leigh's encephalopathy, Huntington disease, Parkinson disease, Alzheimer disease, MNGIE, chronic progressive external ophthalmoplegia (CPEO), MERRF syndrome, Leber's disease and diabetes mellitus.

5. The pharmaceutical agent according to any one of claims 1 to 4, wherein the dose of the agent is 10 to 1,000 mg/kg per day.

6. The pharmaceutical agent according to claim 5, wherein the dose of the agent is 100 to 500 mg/kg per day.

7. The pharmaceutical agent according to any one of claims 1 to 6, wherein the dosage form of the agent is any of tablets, granules, powders and injections.

8. A method for preventing, ameliorating and/or therapeutically treating mitochondrial disorders, comprising a step of ingesting or administering alanine to biological organisms.

9. The method according to claim 8, wherein said ingestion or administration is carried out in the form of pharmaceutical agent according to any one of claims 1 to 7.

10. Use of alanine in producing pharmaceutical agents for mitochondrial disorders.

11. Use according to claim 10, wherein the use in producing the pharmaceutical agent is in the form of pharmaceutical agent according to any one of claims 1 to 7.

## Fig. 1

## Fig. 2

Fig. 3

# INTERNATIONAL SEARCH REPORT

International application No.

PCT/JP03/01462

A. CLASSIFICATION OF SUBJECT MATTER
Int.Cl⁷ A61K31/198, 9/08, 9/14, 9/16, 9/20, A61P3/10, 25/00,
25/10, 25/14, 25/16, 25/28, 27/02, 43/00

According to International Patent Classification (IPC) or to both national classification and IPC

B. FIELDS SEARCHED

Minimum documentation searched (classification system followed by classification symbols)
Int.Cl⁷ A61K31/198, 9/08, 9/14, 9/16, 9/20

Documentation searched other than minimum documentation to the extent that such documents are included in the fields searched

Electronic data base consulted during the international search (name of data base and, where practicable, search terms used)
CA(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>Y | WO 99/53914 A1 (Ajinomoto Co., Inc.),<br>28 October, 1999 (28.10.99),<br>Particularly, page 3, lines 11 to 13<br>& EP 1072264 A1 | 1-3<br>4-7,10,11 |
| Y | JP 2001-521002 A (Cornell Research Foundation,<br>Inc.),<br>06 November, 2001 (06.11.01),<br>Full text<br>& WO 99/21565 A1 | 4-7,10,11 |

☐ Further documents are listed in the continuation of Box C.   ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>20 March, 2003 (20.03.03) | Date of mailing of the international search report<br>08 April, 2003 (08.04.03) |
|---|---|
| Name and mailing address of the ISA/<br>Japanese Patent Office | Authorized officer |
| Facsimile No. | Telephone No. |

Form PCT/ISA/210 (second sheet) (July 1998)

20

## INTERNATIONAL SEARCH REPORT

International application No.

PCT/JP03/01462

**Box I    Observations where certain claims were found unsearchable (Continuation of item 2 of first sheet)**

This international search report has not been established in respect of certain claims under Article 17(2)(a) for the following reasons:

1. ☒ Claims Nos.: 8, 9
   because they relate to subject matter not required to be searched by this Authority, namely:
   The inventions as set forth in claims 8 and 9 pertain to methods for treatment of the human body by therapy.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box II    Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**    ☐    The additional search fees were accompanied by the applicant's protest.

☐    No protest accompanied the payment of additional search fees.

Form PCT/ISA/210 (continuation of first sheet (1)) (July 1998)

SEQUENCE LISTING

<110> Ajinomoto Co., Inc.

<120> PHARMACEUTICAL AGENT FOR MITOCHONDRIAL DISORDERS

<130>

<140>
<141>

<150> JP 2002-036468
<151> 2002-02-14

<160> 26

<170> PatentIn Ver. 2.1

<210> 1
<211> 20
<212> DNA
<213> Artificial Sequence

<220>
<223> Description of Artificial Sequence: 5' terminal of
      Rattus norvegicus mRNA for NAD+specific isocitrate
      dehydrogenase b-subunit, partial cds

<400> 1
tcagcttcca acatgctacg                                                    20

<210> 2
<211> 20
<212> DNA
<213> Artificial Sequence

<220>
<223> Description of Artificial Sequence: 3' terminal of
      Rattus norvegicus mRNA for NAD+specific isocitrate
      dehydrogenase b-subunit, partial cds

<400> 2
cttgccaacc ttgatcacct                                                    20

<210> 3
<211> 20
<212> DNA
<213> Artificial Sequence

<220>
<223> Description of Artificial Sequence: 5' terminal of
      NAD (H)-specific isocitrate dehydrogenase gamma
      subunit

<400> 3
tgggtgatgg actcttcctc                                    20


<210> 4
<211> 20
<212> DNA
<213> Artificial Sequence

<220>
<223> Description of Artificial Sequence: 3' terminal of
      NAD (H)-specific isocitrate dehydrogenase gamma
      subunit

<400> 4
gctgcattgt tgtgttgtcc                                    20


<210> 5
<211> 20
<212> DNA
<213> Artificial Sequence

<220>
<223> Description of Artificial Sequence: 5' terminal of
      Fumarate hydratase

<400> 5
gccaagactg cacacaagaa                                    20


<210> 6
<211> 20
<212> DNA
<213> Artificial Sequence

<220>
<223> Description of Artificial Sequence: 3' terminal of
      Fumarate hydratase

<400> 6
cttgggtttc acccactcat                                    20

<210> 7
<211> 20
<212> DNA
<213> Artificial Sequence

<220>
<223> Description of Artificial Sequence: 5′ terminal of
      Cytochrome c oxidase subunit VIa (liver)

<400> 7
tggaccctga ctcttgtgtg                                        20


<210> 8
<211> 20
<212> DNA
<213> Artificial Sequence

<220>
<223> Description of Artificial Sequence:  3′ terminal
      of Cytochrome c oxidase subunit VIa (liver)

<400> 8
aagggatgga ggagcaaagt                                        20


<210> 9
<211> 20
<212> DNA
<213> Artificial Sequence

<220>
<223> Description of Artificial Sequence: 5′ terminal of
      Cytochrome c oxidase subunit VIIa 3

<400> 9
gttcagtagt cgcggttggt                                        20


<210> 10
<211> 20
<212> DNA
<213> Artificial Sequence

<220>
<223> Description of Artificial Sequence: 3′ terminal of
      Cytochrome c oxidase subunit VIIa 3

<400> 10
gaagtggtgc tgatggtcct                                        20

<210> 11
<211> 20
<212> DNA
<213> Artificial Sequence

<220>
<223> Description of Artificial Sequence: 5' terminal of
Rattus norvegicus liver cytochrome c oxidase
subunit VIII (COX-VIII) mRNA, 3' end of cds

<400> 11
ttcctgcttc gtgtgttgtc                                    20


<210> 12
<211> 20
<212> DNA
<213> Artificial Sequence

<220>
<223> Description of Artificial Sequence: 3' terminal of
Rattus norvegicus liver cytochrome c oxidase
subunit VIII (COX-VIII) mRNA, 3' end of cds

<400> 12
tcaaaggatg agggaagacg                                    20


<210> 13
<211> 20
<212> DNA
<213> Artificial Sequence

<220>
<223> Description of Artificial Sequence: 5' terminal of
Rattus norvegicus mRNA for cytochrome C oxidase
assembly protein COX17, complete cds

<400> 13
tgtgcgatcg ttactgcttt                                    20


<210> 14
<211> 20
<212> DNA
<213> Artificial Sequence

<220>

<223> Description of Artificial Sequence: 3′ terminal of
      Rattus norvegicus mRNA for cytochrome C oxidase
      assembly protein COX17, complete cds

<400> 14
agggcttcag aggcttcttc                                          20


<210> 15
<211> 20
<212> DNA
<213> Artificial Sequence

<220>
<223> Description of Artificial Sequence: 5′ terminal of
      Cytochrome b5, outer mitochondrial membrane
      isoform

<400> 15
ccatcgtggg tgctattctt                                          20


<210> 16
<211> 20
<212> DNA
<213> Artificial Sequence

<220>
<223> Description of Artificial Sequence: 3′ terminal of
      Cytochrome b5, outer mitochondrial membrane
      isoform

<400> 16
aggagatgtg ctccgacact                                          20


<210> 17
<211> 20
<212> DNA
<213> Artificial Sequence

<220>
<223> Description of Artificial Sequence: 5′ terminal of
      ATP synthase subunit d

<400> 17
aacgctctga agtcctggaa                                          20


<210> 18

<211> 20
<212> DNA
<213> Artificial Sequence

<220>
<223> Description of Artificial Sequence: 3' terminal of
     ATP synthase subunit d

<400> 18
gtccacattg gccctgtagt                                          20


<210> 19
<211> 20
<212> DNA
<213> Artificial Sequence

<220>
<223> Description of Artificial Sequence: 5' terminal of
     ATP synthase, H+transporting, mitochondrial FO
     complex, subunit c (subunit 9), isoform 1

<400> 19
aaaaatgcag accacgaagg                                          20


<210> 20
<211> 20
<212> DNA
<213> Artificial Sequence

<220>
<223> Description of Artificial Sequence: 3' terminal of
     ATP synthase, H+transporting, mitochondrial FO
     complex, subunit c (subunit 9), isoform 1

<400> 20
ctactcagga gggaggcaga                                          20


<210> 21
<211> 20
<212> DNA
<213> Artificial Sequence

<220>
<223> Description of Artificial Sequence: 5' terminal of
     Rattus norvegicus (clone gamma-3) ATP synthase
     gamma-subunit (ATP5c) mRNA, 3' end cds

<400> 21
gctgacgtgc tgcagaatta                                                    20


<210> 22
<211> 20
<212> DNA
<213> Artificial Sequence

<220>
<223> Description of Artificial Sequence: 3' terminal of
     Rattus norvegicus (clone gamma-3) ATP synthase
     gamma-subunit (ATP5c) mRNA, 3' end cds

<400> 22
atcctggcac tctgctcact                                                    20


<210> 23
<211> 20
<212> DNA
<213> Artificial Sequence

<220>
<223> Description of Artificial Sequence: 5' terminal of
     2-oxoglutarate carrier

<400> 23
tttcttcagc ctgtggaagg                                                    20


<210> 24
<211> 20
<212> DNA
<213> Artificial Sequence

<220>
<223> Description of Artificial Sequence: 3' terminal of
     2-oxoglutarate carrier

<400> 24
gacgcttgta ggccttgttc                                                    20


<210> 25
<211> 20
<212> DNA
<213> Artificial Sequence

<220>

```
<223> Description of Artificial Sequence: 5' terminal of
      β-actin

<400> 25
aaatgcttct aggcggactg                                          20


<210> 26
<211> 20
<212> DNA
<213> Artificial Sequence

<220>
<223> Description of Artificial Sequence: 3' terminal of
      β-actin

<400> 26
aaagccatgc caaatgtctc                                          20
```